Europäisches Patentamt

European Patent Office

Office européen des brevets

(19)

(11) Publication number: **0 036 699**
**B2**

# (12) NEW EUROPEAN PATENT SPECIFICATION

(45) Date of publication of the new patent
specification: 02.09.87

(51) Int. Cl.⁴: **C 12 P 7/62, C 08 G 63/72**

(21) Application number: 81200352.3

(22) Date of filing: 14.02.80

(60) Publication number of the earlier application in
accordance with Art. 76 EPC: **0 015 123**

(54) Extraction of poly-beta-hydroxybutyric acid.

(30) Priority: 21.02.79 GB 7906076
21.02.79 GB 7906077
08.05.79 GB 7915858

(43) Date of publication of application:
30.09.81 Bulletin 81/39

(45) Publication of the grant of the patent:
02.02.83 Bulletin 83/05

(45) Mention of the opposition decision:
02.09.87 Bulletin 87/36

(84) Designated Contracting States:
BE CH DE FR GB IT LU NL

(56) References cited:
EP-A-0 014 490
EP-A-0 015 669

MACROMOLECULES, vol. 9, no. 5, September-
October 1976, pages 774-780 S. AKITA et al.:
"Solution properties of poly(D-Beta-
hydroxybutyrate). I. Biosynthesis and
characterization"

(73) Proprietor: IMPERIAL CHEMICAL INDUSTRIES
PLC
Imperial Chemical House Millbank
London SW1P 3JF (GB)

(72) Inventor: Holmes, Paul Arthur
39 Skottowe Crescent Great Ayton
Middlesbrough Cleveland (GB)
Inventor: Wright, Leonard Feredrick
8 Blairmore Gardens Eaglescliffe
Stockton-on-Tees Cleveland (GB)

(74) Representative: Denerley, Paul Millington, Dr.
et al
Imperial Chemical Industries PLC Legal
Department: Patents P.O. Box 6, Bessemer Road
Welwyn Garden City Hertfordshire AL7 1HD (GB)

(56) References cited:
Industrial & Engineering Chemistry April 1947,
Vol. 39, No. 4, page 519

Courier Press, Leamington Spa, England.

## Description

This invention relates to an extraction process and in particular to a process of extracting poly-β-hydroxybutyric acid, hereinafter referred to as PHB from microbial cells.

It has been known since the 1920's that many microorganisms are capable of accumulating granules of PHB within their cells as an energy reserve material. It has been proposed in US—A—3 107 172 to dry such PHB containing bacterial cells, for example by spray drying, and to use the resultant dried cells as moulding composition. Suggestions have also been made to extract PHB from bacterial cells and to use it as a plastic material, but methods so far disclosed have not been economically acceptable.

In order to extract the PHB, it is generally necessary to contact the bacterial cells with a solvent in which PHB is soluble to leach out the PHB from the remainder of the bacterial cell material. Some bacteria, for example members of the genus *Azotobacter* readily yield up their PHB to the extraction solvent, whereas other bacteria, e.g. *Pseudomonadaceae* have more robust cells and require a cell disruption step prior to contact with the extraction solvent.

Methods of extraction previously proposed have included the steps of harvesting the bacterial cells from the aqueous fermentation medium, e.g. by centrifugation, to give a mass of wet cells which are then contacted with acetone to effect drying and cell breakage. After removal of the acetone, the PHB is extracted with a suitable solvent, e.g. pyridine (US—A—3 036 959) or a dichloroethane/ethanol mixture (US—A—3 044 942). Such methods have the advantage that in addition to effecting drying and cell breakage, acetone also extracts lipids and pigments (if any) which would otherwise contaminate the product. However treatment of a mass of wet cells with acetone to effect drying and cell breakage is not economic on a large scale.

Another method is described in US—A—3 275 610 wherein a dispersion of bacterial cells in water is subjected to ultrasonic vibration to rupture the cells followed by centrifugation and drying before extraction with a solvent such as chloroform. After separation of the PHB from the chloroform solution, the PHB is washed to extract lipids therefrom.

It has also been proposed in US—A—4 101 533 to extract PHB from dried cells, or directly from a wet mass of cells harvested from the fermentation medium by centrifugation, by heating the cells with certain cyclic carbonate solvents.

We have found that it is possible to extract PHB directly from the aqueous cell suspension produced by fermentation, preferably after some concentration, by contact with 1,2-dichloroethane as the extraction solvent with, where necessary, a cell disruption step, e.g. milling, prior to contact with the solvent. 1,2-Dichloroethane is not normally considered to be a solvent for PHB because PHB, after separation from bacterial cells, does not readily, or completely, dissolve in 1,2-dichloroethane. Thus, whereas the PHB solution separated from the bacterial cells residue appears to be a single phase, a solution made by redissolving PHB in 1,2-dichloroethane after precipitation is, except when very dilute, pearly in appearance, and PHB that has been precipitated and dried does not readily re-dissolve in that solvent. It is therefore surprising that such a solvent should be effective for extracting PHB from cells.

To avoid undue uptake by the solvent of non-PHB material, particularly lipids and pigment (if any), present in the bacterial cell, the extraction temperature should be below 40°C. Not only does such non-PHB material contaminate the product and so present purification difficulties but also the co-extraction of lipids may tend to result in the formation of a relatively stable emulsion between the solvent and aqueous phases rendering separation thereof difficult. With such a direct extraction process, a separate lipid extraction step prior to contact with the PHB extraction solvent is generally not practical as the solvents that extract lipids would need to be removed, together with the lipids, prior to contact with the PHB extraction solvent and, because the more effective lipid solvents tend to be water miscible, such removal of the lipid solution presents practical difficulties.

Accordingly the present invention provides a process for the extraction of PHB from an aqueous suspension of PHB-containing bacterial cells comprising contacting said suspension with 1,2-dichloroethane at a temperature below 40°C and then separating the PHB containing solvent phase from the bacterial cell residue.

We have found that, when extracting the PHB directly from the aqueous cell suspension with 1,2-dichloroethane, the PHB can be extracted from some baceria without the need for a separate cell disruption step. Thus members of the genera *Azotobacter* and *Alcaligenes* readily yield up their PHB to the 1,2-dichloroethane solvent such that the extraction step requires only agitation of the cell suspension with the solvent. The agitator preferably includes adjacent surfaces in relative movement thus providing mild shearing. A Silverson (RTM) blender may be used to effect mixing. More robust bacteria, e.g. *Pseudomonadaceae* require a distinct step of cell disruption. This disruption can be carried out by shearing the aqueous cell suspension, for example by homogenisation, bead-milling, roller-milling, or French pressing. Other methods of cell disruption include osmotic shock, sonic or ultrasonic vibration, and enzyme cell-wall lysis. Treatment with hypochlorite to destroy the bacterial cell membrane chemically could be used but preferably is not because it usually causes severe degradation of the PHB.

As mentioned hereinbefore, to avoid undue uptake of non-PHB material by the 1,2-dichloroethane, the extraction temperature should be below 40°C; the extraction temperature is preferably in the range 10 to 40°C.

2

The weight of PHB extraction solvent used is preferably 10 to 100 times the cell dry weight. The use of smaller amounts of solvent may reduce the extraction efficiency of crude PHB and may give solutions of excessive viscosity while the use of larger amounts is uneconomic. The amount of solvent is preferably such that the extracted solution contains 0.5 to 5%, particularly 1 to 2% PHB by weight.

The contacting time for extraction should be a compromise to give adequate extraction without being uneconomically lengthy.

The PHB is extracted by contacting an aqueous suspension of the bacterial cells with 1,2-dichloroethane. The bacterial cell residue may be removed by separating the 1,2-dichloroethane phase (which contains the dissolved PHB) from the aqueous phase: the bacterial residue remains suspended in the aqueous phase. It is sometimes difficult to separate the 1,2-dichloroethane phase from the aqueous medium as a stable emulsion may be formed. While in some cases centrifugation will facilitate separation of such emulsions, centrifugation is not always successful and anyway is not attractive in large scale operation.

We have found that separation of the solvent and aqueous phases may be facilitated by adjustment of the pH of the aqueous phase, prior to contact with the 1,2-dichloroethane, to within 0.5 units of pH of the isoelectric point of the bacterial cells. As will be mentioned hereinafter, for some bacterial cells, it is desirable to disrupt the cells prior to contact of the aqueous cell suspension with the 1,2-dichloroethane. The isoelectric point of the disrupted cells may well differ from that of the unbroken cells. Where the cells are disrupted the pH should be adjusted to within 0.5 units of pH of the isoelectric point of the disrupted cells. While it is possible to add sufficient of a pH modifying material to the suspension of cells prior to cell disruption so that, on disruption the pH is within 0.5 units of the isoelectric point of the disrupted cells, it is preferred to adjust the pH after the step of cell disruption.

The isoelectric point of a bacterial cell is the pH value at which the cell possesses no set positive or negative charge, i.e. it is electrically neutral. The isoelectric point may be determined by particle electrophoresis measurements at differing pH values and hence the pH at which the net charge is zero is determined.

Generally for PHB-accumulating bacteria the isoelectric point, both for whole cells and for disrupted cells, is within the pH range 4 to 5.5; however the pH of the aqueous medium during fermentation will generally be within the range 6 to 8, the optimum depending on the nature of the bacteria used and the other fermentation conditions. As indicated above the pH of the aqueous medium should be adjusted to within 0.5 units of pH of the isoelectric point. Preferably it is adjusted to within 0.25 pH units of the isoelectric point and, more particularly, to a pH within the range of the isoelectric point to 0.25 pH units above the isoelectric point.

After separation of the PHB-containing extraction solvent from the bacterial cell residue, the PHB solution may be further filtered, if desired, to remove any suspended bacterial fragments. Such filtration is preferably conducted using a filter, e.g. a glass fibre filter, having a pore size of less than 5 μm, preferably less than 2 μm.

The separated PHB-containing solution can be used directly, preferably after filtration, for making solvent cast articles such as coatings, films or fibres or the solution may be treated further to separate solid PHB, for example by evaporation of the solvent or by precipitation by addition of the PHB-containing solution to a liquid in which PHB is insoluble and with which the solvent is miscible. Examples of suitable liquids include petroleum ether and methanol/water mixtures. The PHB may be purified, if desired, by washing with methanol or acetone.

After extraction of the PHB, the bacterial cell residues may be further refined for other uses, e.g. as a feedstuff or fertilizer.

Any bacteria that are capable of accumulating PHB may be used to produce the PHB-containing bacterial cells. A paper by Senior et al in Advances in Microbial Physiology 1973, *10*, 203—266 lists the bacteria published up to June 1972 and others are described in US Patent 3 072 538 (*Rhizobium* mutants) and UK Patent 1 535 632 (especially mutants of *Alcaligenes eutrophus, Bacillus megaterium, Zoogloea ramigera,* and *Mycoplana rubra*). Among the preferred bacteria are *Azotobacter,* especially *chroococcum, Alcaligenes,* especially *eutrophus,* and the *Pseudomonadaceae,* especially *Pseudomonas* AM1 and *Methylobacterium organophilum.*

Among such bacteria are those capable of metabolising one or more of a variety of substrates, for example carbohydrates, ethanol, methanol, polyhydric alcohols, carbon dioxide/hydrogen, and carboxylic acids, and, according to the substrate used, may grow aerobically or anaerobically. The invention is of particular utility in separating PHB from bacterial cells of the *Pseudomonadaceae* grown under aerobic fermentation conditions on an alcohol, particularly methanol, substrate. The invention is also of particular utility in separating PHB from *Azotobacter* grown on a water soluble carbohydrate such as sucrose or glucose.

The cell suspension produced by the fermentation process will typically contain 20 to 55 g $l^{-1}$ biomass solids. The cell suspension preferably has a concentration of 5 to 15% by weight biomass solids. The cell suspension is preferably concentrated, e.g. by centrifugation, to within this range where this is necessary. (The cell suspension as produced in the fermentation process may already have a concentration within this range: however even in such cases some concentration may be desirable).

3

**0 036 699**

The invention is illustrated by the following examples in which all percentages are by weight. In the examples yields are calculated as

$$\frac{\text{weight of crude PHB recovered}}{\text{dry weight of cells used}} \times \frac{100}{\text{\% PHB in cells}} \times 100$$

Also the NCIB numbers refer to the numbers designated to cultures deposited at the National Collection of Industrial Bacteria, Torry Research Station, Aberdeen, Scotland.

Example 1

A culture of *Azotobacter chroococcum* (NCIB 9125) was produced by fermentation of glucose in an aqueous medium under carbon-excess, oxygen-limitation, and minimal salts concentration conditions. The cell suspension contained 20 g $l^{-1}$ biomass solids, the PHB content of which was 40%. The suspension was then concentrated to a cell cream containing 80 g $l^{-1}$ biomass solids by centrifugation.

500 ml of the cell cream was mixed with 1000 ml of 1,2-dichlorethane at 20°C and blended for 10 minutes by means of a Silverson (RTM) blender, model L2R. Owing to energy dissipation the temperature rose to 40°C. The resulting emulsion was cooled and then centrifuged at 13000 g for 15 minutes at 15°C. The upper layer of aqueous solution and cell debris was decanted off. The lower layer, PHB-dissolved, PHB-dissolved in 1,2-dichloroethane, was poured slowly with vigorous stirring into 5000 ml of a methanol/water mixture (4 volumes methanol:l volume water).

The precipitate (purity 97.5%) was collected on a silver, washed five times with 1000 ml of methanol and dried *in vacuo* at 50°C. It was analysed chemically and found to have the following % composition: C 55.8, H 7.0, O 37.0, N <0.2 (theoretical C 55.9, H 6.9, O 37.2). This corresponds to a purity of over 99.5%. The solid PHB has a fibrous consistency. The yield was 14.7 g (92%).

By way of comparison, when the above procedure was repeated using chloroform as the extraction solvent, while the yield was 16.6 g, the purity (before methanol washing) was only 88.6%.

Example 2

An aqueous suspension of cells of Pseudomonas AM 1 (NCIB 9133) made by fermentation of methanol in an aqueous medium and containing 20 g $l^{-1}$ total biomass solids of which 28% was PHB was extracted by contact with 1,2-dichloroethane by the technique of Example 1 save that, after concentration to a cell cream containing 120 g $l^{-1}$ biomass solids, the cells were disrupted by milling in a Dynomill at a throughput rate of 15 l $hr^{-1}$ at an inlet temperature of 20°C and an outlet temperature of 40°C.

The PHB produced had a purity (before methanol washing) of 93.2% and the yield was 77%.

Example 3

Example 2 was repeated using an aqueous suspension of cells of *Methylobacterium organophilum* (NCIB 11483) concentrated to a cell cream of 120 g $l^{-1}$ biomass solids by centrifugation. The yield of crude PHB (purity 93.2%) was 63.8%. The level of this yield was partly as a result of the fact that the separated lower layer had a volume of only 750 ml, i.e. only 75% of the 1,2-dichloroethane was separated from the emulsion. If all the 1,2-dichloroethane had been separated, calculation shows that the yield of PHB would have been about 85%.

Example 4

Example 1 was repeated except that after mixing the cell cream (which had a pH of 7 and had been stored for a few weeks after fermentation) and 1,2-dichloroethane in the Silverson blender, the emulsion was allowed to settle under natural gravity for 30 minutes instead of cooling and separation by centrifugation. During this time the emulsion separated into an upper aqueous layer containing the cell debris and a lower layer of a solution of PHB in 1,2-dichloroethane. The two layers were separated by decanting. The recovered lower layer had a volume of only 170 ml. Even after standing for 1 hour the volume of the separated lower layer was only 420 ml. To obtain rapid separation with this system (at pH 7) it was necessary to cool the emulsion and centrifuge it for 15 minutes at 15°C.

Example 5

Example 4 was repeated but the pH of the cell cream was adjusted to various values before contacting the suspension with 1,2-dichloroethane. The amount of 1,2-dichloroethane solution separating on standing for 30 and 60 minutes was as follows

4

Volume of 1,2-dichloroethane separated (ml)

| pH | after 30 minutes | after 60 minutes |
|---|---|---|
| 4 | 0 | 30 |
| 4.5 | 410 | — |
| 5 | 650 | 770 |
| 5.5 | 640 | — |
| 6 | 290 | 700 |
| 7 | 170 | 420 |
| 8 | 60 | 300 |
| 9 | 50 | 270 |
| 10 | 30 | — |

The isoelectric point of the cell cream was also determined and found to be at pH 5.

Example 6

Example 4 was repeated with a freshly fermented sample of an *Azotobacter chroococcum* (NCIB 9125) cell cream of isoelectric point at pH 5 in which the cells contained about 40% PHB. The pH was adjusted to 5 prior to contacting the cell cream with 1,2-dichloroethane. The aqueous and 1,2-dichloroethane layers separated rapidly under natural gravity and after only 10 minutes 850 ml of the 1,2-dichloroethane layer had separated and was recovered. The PHB was precipitated therefrom and washed, as in Example 1. The yield of PHB (purity 99.5%) was 84%.

Example 7

An aqueous suspension of cells of Pseudomonas AM 1 (NCIB 9133) was made by aerobic fermentation of methanol in an aqueous medium at pH 7.0. The suspension contained 20 g $l^{-1}$ total biomass solids of which 30% was PHB. The suspension was concentrated by means of a centrifuge to a cell cream containing 80 g $l^{-1}$ biomass solids. The cells were disrupted by milling the cell cream in a Dynomill at a throughput rate of 12 l $hr^{-1}$ at an inlet temperature of 20°C and an outlet temperature of 40°C. The isoelectric point of the resultant broken cells was determined and found to be pH 4.75.

The pH of the suspension of broken cells was adjusted to 4.75 and 500 ml of the suspension was added to 1 litre of 1,2-dichloroethane. The mixture was mixed for 10 minutes using a Silverson (RTM) blender, model L2R at a low mixing speed. The resultant emulsion was then allowed to settle. After 10 minutes 800 ml of 1,2-dichloroethane had separated. The PHB was then separated from the 1,2-dichloroethane by the procedure of Example 1. The yield of PHB was 78%.

When this experiment was repeated but without any pH adjustment, on mixing the suspension of broken cells and 1,2-dichloroethane, a relatively stable emulsion was formed that could be separated only after centrifugation.

Example 8

To compare the effectiveness of various solvents as extraction solvents 500 ml of *Azotobacter chroococcum* (NCIB 9125) cell cream of biomass content 50 g $l^{-1}$ and pH 5.0 was poured at 20°C into 1 litre of the solvent under examination. The resulting mixture was mixed in a Silverson blender model L2R for 10 minutes and then allowed to separate under gravity. The amount of solvent separated after 30 and 60 minutes was measured. After separation, the solvent layer, herein termed "syrup", was syphoned off and its solids content determined by evaporation of a sample to dryness. The PHB content of the syrup was also determined by precipitation into a water/methanol mixture as in Example 1.

The results are shown in the following table.

| | | % solvent recovered | | % solids content of syrup | |
|---|---|---|---|---|---|
| Solvent | | 30 min | 60 min | total | PHB |
| 1,2-dichloroethane | | 55 | 75 | 1.15 | 0.89 |
| chloroform* | | 0 | 0 | 1.27 | 0.92 |
| dichloromethane* | | 12 | 35 | 1.51 | 0.95 |
| 1,1,1-trichlorethane | | 45 | 60 | 0.8 | ND |
| 1,1,2-trichloroethylene | | 45 | 50 | 0.12 | ND |
| 1,1,2,2-tetrachloroethane | | 85 | 90 | 0.01 | ND |
| pyridine* | | 0 | 0 | 0.01 | ND |
| 1,2-propylene carbonate* | | 8 | 15 | 0.01 | ND |

ND = none detected

* in view of the low separations on standing, the emulsion was centrifuged to obain the samples for determination of the total solids and PHB contents.

To illustrate the solubility characteristics of PHB in various solvents, re-solution of the precipitated PHB obtained by the cold extraction with chloroform, dichloromethane and 1,2-dichloroethane was attempted by mixing the precipitated PHB after drying with a fresh quantity of the original cold solvent. The precipitated PHB redissolved in cold chloroform or dichloromethane but was not soluble in cold 1,2-dichloroethane.

## Claims

1. A process for the extraction of poly β-hydroxybutyric acid (PHB) from an aqueous suspension of bacterial cells containing PHB comprising contacting said suspension with 1,2-dichloroethane at a temperature below 40°C and then separating the PHB containing solvent phase from the bacterial cell residue.

2. A process according to claim 1 in which the aqueous suspension of the cells is subjected to a cell disruption step prior to contact of the cells with 1,2-dichloroethane.

3. A process according to claim 1 or claim 2 wherein the pH of the aqueous suspension is adjusted to within 0.5 units of pH of the isoelectric point of the cells, or of the disrupted cells if the cells are subjected to a cell disruption step, prior to contact with the 1,2-dichloroethane.

4. A process according to any one of claims 1 to 3 in which the weight of 1,2-dichloroethane is 10 to 100 times the cell dry weight.

5. A process according to any one of claims 1 to 4 in which the PHB is separated from the 1,2-dichloroethane by precipitation into a liquid in which PHB is insoluble and with which 1,2-dichloroethane is miscible.

## Patentansprüche

1. Verfahren zur Extraktion von Poly-β-hydroxybuttersäure (PHB) aus einer wäßrigen Suspension von PHB enthaltenden Bakterienzellen, bei dem die erwähnte Suspension bei einer unterhalb von 40°C liegenden Temperatur mit 1,2-Dichlorethan in Berührung gebracht und die PHB enthaltende Lösungsmittelphase dann von dem Bakterienzellenrückstand abgetrennt wird.

2. Verfahren nach Anspruch 1, bei dem die wäßrige Suspension der Zellen vor dem Inberührungbringen der Zellen mit 1,2-Dichlorethan einem Schritt unterzogen wird, in dem die Zellen zerrissen werden.

3. Verfahren nach Anspruch 1 oder Anspruch 2, bei dem der pH der wäßrigen Suspension vor den Inberührungbringen mit dem 1,2-Dichlorethan auf einen Wert eingestellt wird, der sich von dem isoelektrischen Punkt der Zellen oder der zerrissenen Zellen, falls die Zellen einem Schritt unterzogen werden, in dem sie zerrissen werden, um bis zu 0,5 pH-Einheiten unterschiedet.

4. Verfahren nach einem der Ansprüche 1 bis 3, bei dem das Gewicht des 1,2-Dichlorethans das 10- bis 100 fache des Trockengewichts der Zellen beträgt.

5. Verfahren nach einem der Ansprüche 1 bis 4, bei dem die PHB durch Ausfällung in eine mit 1,2-Dichlorethan mischbare Flüssigkeit, in der PHB unlöslich ist, von dem 1,2-Dichlorethan abgetrennt wird.

# 0 036 699

**Revendications**

1. Procédé pour l'extraction d'acide poly-β-hydroxybutyrique (PHB) à partir d'une suspension aqueuse de cellules bactériennes contenant du PHB, consistant à mettre les cellules bactériennes en contact avec du 1,2-dichloréthane à une température inférieure à 40°C et à séparer ensuite la phase solvant contenant le PHB du résidu de cellules bactériennes.

2. Procédé suivant la revendication 1, caractérisé en ce qu'on soumet la suspension aqueuse des cellules à une étape de rupture des cellules avant la mise en contact de ces cellules avec le 1,2-dichloréthane.

3. Procédé suivant la revendication 1 ou la revendication 2, caractérisé en ce qu'on règle le pH de la suspension aqueuse à une valeur qui diffère de moins de 0,5 unité de pH du point isoélectrique des cellules ou des cellules rompues si les cellules sont soumises à une étape de rupture des cellules avant la mise en contact avec le 1,2-dichloréthane.

4. Procédé suivant l'une quelconque des revendications 1 à 3, caractérisé en ce que le poids de 1,2-dichloréthane représente de 10 à 100 fois le poids à sec des cellules.

5. Procédé suivant l'une quelconque des revendications 1 à 4, caractérisé en ce qu'on sépare le PHB du 1,2-dichloréthane par précipitation dans un liquide dans lequel le PHB est insoluble et avec lequel le 1,2-dichloréthane est miscible.

7